# EUROPEAN PATENT SPECIFICATION

(11) **EP 2 186 482 B1**
(45) Date of publication and mention of the grant of the patent: **18.01.2012**
(21) Application number: 09005620.1
(22) Date of filing: 22.04.2009
(51) Int. Cl.: A61B 8/08

(54) **Ultrasound system and method providing acoustic radiation force impulse imaging with high frame rate**
Ultraschallsystem und -verfahren zur Bereitstellung von Schallstrahlungs-Druckimpulsbildern mit hoher Bildfrequenz
Système ultrasonore et procédé pour fournir une imagerie à impulsion de force à rayonnement acoustique avec une fréquence d'image élevée

(30) Priority: 18.11.2008 KR 20080114609
(43) Date of publication of application: 19.05.2010
(73) Proprietor: Samsung Medison Co., Ltd., Nam-myun Hongchun-gun Kangwon do (KR)
(72) Inventor: Joo, Jongho, Seoul 137-848 (KR); Kim, Jong-Sik, Seoul 143-757 (KR)
(74) Representative: Schmid, Wolfgang

(56) References cited:
- WO-A1-2006/042201
- WO-A2-02/43564
- US-A1- 2004 068 184
- US-A1- 2005 215 899
- BERCOFF J ET AL: "SUPERSONIC SHEAR IMAGING: A NEW TECHNIQUE FOR SOFT TISSUE ELASTICITY MAPPING" IEEE TRANSACTIONS ON ULTRASONICS, FERROELECTRICS AND FREQUENCY CONTROL, IEEE, US, vol. 51, no. 4, 1 April 2004 (2004-04-01), pages 396-409, XP001218171 ISSN: 0885-3010

## Description

### BACKGROUND

### Field of the Invention

Embodiments of the present invention relate to an ultrasound system and method, and more particular, to an ultrasound system and method for providing an acoustic radiation force impulse image.

### Description of the Related Art

An ultrasound system denotes a system that may emit ultrasound signals from the body surface of a subject to a selected interior portion of the body and provide images associated with blood flow or a section of soft tissue using information associated with reflected ultrasound signals. The ultrasound system is generally small and inexpensive, and also provides a display in real time. In addition, the ultrasound system has no absorbed dose such as with X rays and the like, and thus is highly stable. The ultrasound system is being widely used together with other image diagnostic apparatuses such as an X-ray diagnostic apparatus, a computerized tomography (CT) scanner, a magnetic resonance image (MRI) apparatus, a nuclear medicine diagnostic apparatus, and the like. In particular, the ultrasound system may display an interior body image in real time and thus is being variously used.

A human tissue has a characteristic of elasticity among various types of characteristics. The elasticity indicates a transformation level of the tissue with respect to a given unit force. As the elasticity increases, the transformation level may decrease. Conversely, as the elasticity decreases, the transformation level may increase. The elasticity may be a unique characteristic of the tissue. That the elasticity of the tissue has changed may mean that a physical property of the tissue has changed. For example, a cancer tissue generally has the elasticity by three times greater than a normal tissue. Such tissue generation may not be observed using a general ultrasound image. Accordingly, when the elasticity of the tissue is observed using ultrasound, it is possible to discern the cancer tissue from the normal tissue.

In US 2005/215899 A1 ultrasound methods and systems for distinguishing ablated tissue from unablated tissue include scanning ablated tissue using Acoustic Radiation Force Impulse (ARFI) imaging. ARFI image data is generated based on the scanning. The image data includes a portion of increased stiffness representing the ablated tissue that is distinguishable from unablated tissue.

### SUMMARY

According to an aspect of the present invention, there is provided an ultrasound method of providing an acoustic radiation force impulse image, the method including: transmitting, to a target tissue, a pushing ultrasound signal for generating of a displacement, using a probe; receiving a response signal from the target tissue in correspondence to a tracking ultrasound signal transmitted via the probe; detecting displacement information associated with the target tissue using the response signal; and generating the acoustic radiation force impulse image based on the displacement information, wherein the transmitting of the pushing ultrasound signal includes simultaneously transmitting the pushing ultrasound signal along a plurality of scan lines that are spaced apart from each other by a predetermined distance, wherein the pushing ultrasound signal is transmitted with respect to different focal points for each of the scan lines and wherein a frequency of the pushing ultrasound signal is variable according to each of the different focal points.

Alternatively there is provided an ultrasound method of providing an acoustic radiation force impulse image, the method including: transmitting, to a target tissue, a pushing ultrasound signal for generating of a displacement, using a probe; receiving a response signal from the target tissue in correspondence to a tracking ultrasound signal transmitted via the probe; detecting displacement information associated with the target tissue using the response signal; and generating the acoustic radiation force impulse image based on the displacement information, wherein the transmitting of the pushing ultrasound signal may include sequentially transmitting the pushing ultrasound signal with respect to a plurality of focal points along a scan line and wherein a frequency of the pushing ultrasound signal may be variable according to each of the focal points.

Also, the probe may include two-dimensionally arranged transducers and the transducers may be classified into a plurality of sections. While the pushing ultrasound signal is being transmitted using a transducer included in a first section among the plurality of sections, the tracking ultrasound signal may be transmitted using a transducer included in a second section among the plurality of sections.

Also, the method may further include transmitting a control command to a cooling device associated with the target tissue in correspondence to transmitting of the pushing ultrasound signal.

According to another aspect of the present invention, there is provided an ultrasound system for providing an acoustic radiation force impulse image, the system including: a transceiving unit to transmit, to a target tissue, a pushing ultrasound signal for generating of a displacement, using a probe, and to receive a response signal from the target tissue in correspondence to a tracking ultrasound signal transmitted via the probe; a detection unit to detect displacement information associated with the target tissue using the response signal; and a generation unit to generate the acoustic radiation force impulse image based on the displacement information.

According to embodiments of the present invention, there may be provided an ultrasound system and method for providing an acoustic radiation force impulse image that may apply a pushing ultrasound signal along a plurality of scan lines and thereby improve a frame rate.

Also, according to embodiments of the present invention, there may be provided an ultrasound system and method for providing an acoustic radiation force impulse image that may apply a pushing ultrasound signal with respect to a plurality of focal points for each single scan line and thereby prevent overheating of a target tissue and may also improve a frame rate.

Also, according to embodiments of the present invention, there may be provided an ultrasound system and method for providing an acoustic radiation force impulse image that may simultaneously apply a pushing ultrasound signal and a tracking ultrasound signal using different transducers and thereby more effectively obtain an acoustic radiation force impulse image.

Also, according to embodiments of the present invention, there may be provided an ultrasound system and method for providing an acoustic radiation force impulse image that may prevent overheating of a target tissue via a cooling device and thereby prevent a degeneration and a necrosis of the target tissue.

### BRIEF DESCRIPTION OF THE DRAWINGS

These and/or other aspects, features, and advantages of the invention will become apparent and more readily appreciated from the following description of exemplary embodiments, taken in conjunction with the accompanying drawings of which:
- FIG 1: is a block diagram illustrating an ultrasound system for providing an acoustic radiation force impulse image according to an embodiment of the present invention;
- FIG 2: is a flowchart illustrating an ultrasound method of providing an acoustic radiation force impulse image according to an embodiment of the present invention;
- FIG 3: illustrates an example of transmitting a pushing ultrasound signal along a plurality of scan lines according to an embodiment of the present invention;
- FIG. 4: illustrates an example of transmitting a pushing ultrasound signal with respect to a plurality of focal points according to an embodiment of the present invention; and
- FIG 5: illustrates an example of transmitting a different ultrasound signal for each transducer section according to an embodiment of the present invention.

### DETAILED DESCRIPTION

Reference will now be made in detail to exemplary embodiments of the present invention, examples of which are illustrated in the accompanying drawings, wherein like reference numerals refer to the like elements throughout. Exemplary embodiments are described below to explain the present invention by referring to the figures.

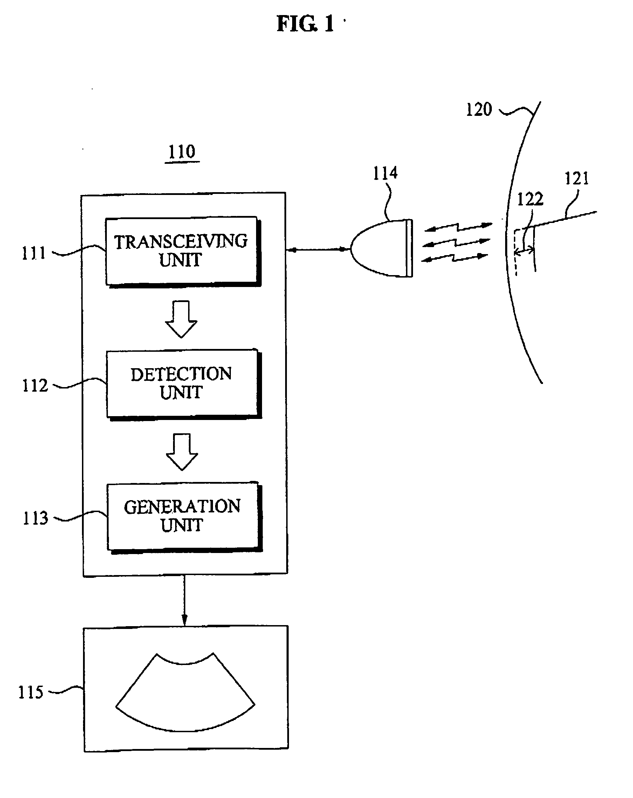

FIG 1 is a block diagram illustrating an ultrasound system 110 for providing an acoustic radiation force impulse image according to an embodiment of the present invention.

As shown in FIG. 1, the ultrasound system 110 may receive a response signal in correspondence to a tracking ultrasound signal that is transmitted to a subject 120 via a probe 114. The response signal denotes a signal that is transmitted or reflected from the subject 120 by the tracking ultrasound signal. Also, the ultrasound system 110 may generate ultrasound image data using the response signal, generate an ultrasound image from the ultrasound image data, and display the ultrasound image via an internal or external display device 115. Here, the ultrasound image may be displayed in either a two-dimensional (2D) form or a three-dimensional (3D) form. Also, the subject 120 may be a human body. The probe 114 may include transducers that transmit and receive the tracking ultrasound signal.

The ultrasound system 110 may transmit a pushing ultrasound signal to the subject 120 via the probe 114. In this case, the pushing ultrasound signal may cause a displacement 122 in a target tissue 121 of the subject 120. The pushing ultrasound signal may be transmitted to the subject 120 prior to transmitting of the tracking ultrasound signal and reception of the response signal.

The ultrasound system 110 may detect displacement information associated with the target tissue 121 using the response signal and generate the acoustic radiation force impulse image based on the displacement information. The displacement information may include a displacement level of the target tissue 121 that is caused by the pushing ultrasound signal.

The acoustic radiation force impulse image may indicate an elasticity level of the target tissue 121. A user may determine a state of the target tissue 121 based on the elasticity level of the target tissue 121. For example, the user may detect a tissue such as a wen having an elasticity less than a general tissue and may also detect a tissue degeneration such as a cancer having an elasticity greater than the general tissue, using the acoustic radiation force impulse image.

As described above, the ultrasound system 110 may provide the acoustic radiation force impulse image of the target tissue 121 together with the general ultrasound image.

The ultrasound system 110 may include a transceiving unit 111, a detection unit 112, and a generation unit 113. Here, the transceiving unit 111 may transmit, to the target tissue 121, a pushing ultrasound signal for generating of a displacement using the. probe 114 and may receive a response signal from the target tissue 11 in correspondence to a tracking ultrasound signal transmitted via the probe 114. The detection unit 112 may detect displacement information associated with the target tissue 121 using the response signal. The generation unit 113 may generate the acoustic radiation force impulse image based on the displacement information. Also, although not shown in FIG 1, the ultrasound system 110 may further include a cooling device to decrease a temperature of the target tissue 121. The transceiving unit 111 may transmit a control command to the cooling device in correspondence to transmitting of the pushing ultrasound signal.

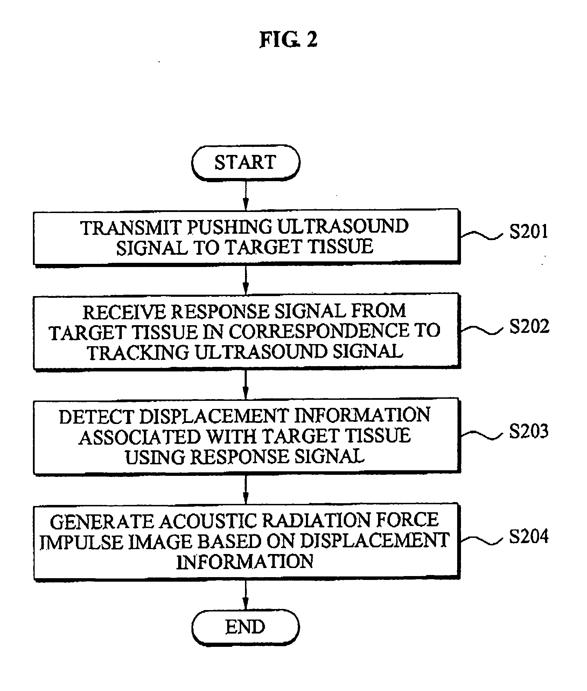

Hereinafter, an operating method of the ultrasound system 110 constructed as above will be further described in detail with reference to FIGS. 2 through 5.

FIG. 2 is a flowchart illustrating an ultrasound method of providing an acoustic radiation force impulse image according to an embodiment of the present invention.

As shown in FIG. 2, the ultrasound method of providing the acoustic radiation force impulse image may be performed via operations S201 through S204. Here, operations S201 and S202 may be performed by the transceiving unit 111. Operation S203 may be performed by the detection unit 112. Operation S204 may be performed by the generation unit 113.

In operation S201, the transceiving unit 111 may transmit, to a target tissue, a pushing ultrasound signal for generating of a displacement using a probe. Here, the pushing ultrasound signal may induce a displacement of the target tissue. Specifically, the target tissue may be moved by the pushing ultrasound signal. This movement may induce the displacement. The displacement of the target tissue may be in inverse proportion to an elasticity of the target tissue. A recovering speed of a tissue may be in proportion to a viscoelasticity of the target tissue.

Generally, a short and strong sound wave may cause a greater displacement in comparison to a long and weak sound wave. Accordingly, examples of the pushing ultrasound signal may include an ultrasound signal that has a single pulse and a great amplitude. Since a maximum output of the ultrasound signal of the ultrasound system 110 is pre-determined, the transceiving unit 111 may generate the pushing ultrasound signal by extending the ultrasound signal of the maximum output as long as possible. Also, according to an embodiment of the present invention, the transceiving unit 111 may generate the pushing ultrasound signal using a sufficiently long color Doppler pulse signal with a great amplitude.

In operation S202, the transceiving unit 111 may receive a response signal from the target tissue in correspondence to a tracking ultrasound signal transmitted via the probe. Here, the tracking ultrasound signal may be used to measure a level of the displacement of the target tissue. The response signal may include information associated with the level of the displacement. For example, the tracking ultrasound signal may include a B mode ultrasound signal. The tracking ultrasound signal may be transmitted to a region of interest (ROI) including the target tissue. The response signal may be reflected from the ROI and thereby be received.

According to an embodiment of the present invention, the transceiving unit 111 may simultaneously transmit the pushing ultrasound signal along a plurality of scan lines that are spaced apart from each other by a predetermined distance.

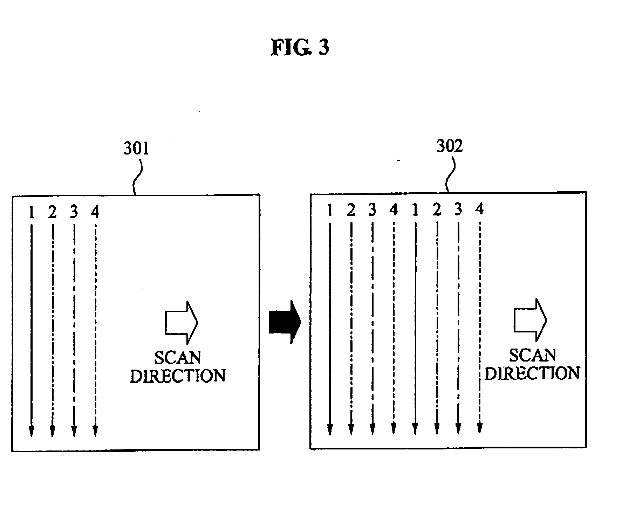

FIG. 3 illustrates an example of transmitting a pushing ultrasound signal along a plurality of scan lines according to an embodiment of the present invention.

As shown in a block 301, the transceiving unit 111 may transmit the pushing ultrasound signal to a target tissue via a single scan line. Also, as shown in a block 302, the transceiving unit 111 may simultaneously transmit the pushing ultrasound signal along the plurality of scan lines that are spaced apart from each other by a predetermined distance. As described above, the transceiving unit 111 may measure a displacement of the target tissue at the plurality of scan lines at one time by simultaneously transmitting the pushing ultrasound signals via the plurality of scan lines. Also, since the transceiving unit 111 may transmit the pushing ultrasound signal via the plurality of scan lines that are spaced apart from each other by the predetermined distance, it is possible to distribute a temperature rise of the target tissue and thereby prevent overheating or damage of the target tissue.

Also, the transceiving unit 111 may transmit the tracking ultrasound signal to the target tissue along the plurality of scan lines and receive a response signal that is reflected from the target tissue in correspondence to transmitting of the tracking ultrasound signal.

According to an embodiment of the present invention, the transceiving unit 111 may sequentially transmit the pushing ultrasound signal to a plurality of focal points along a scan line.

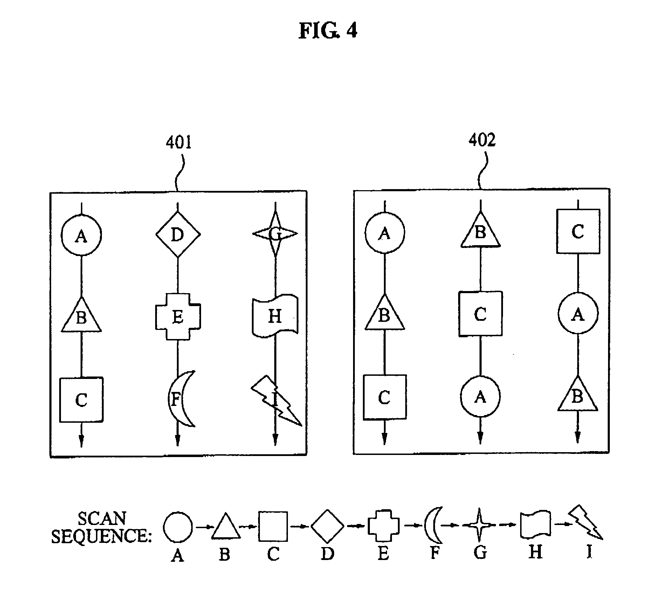

FIG 4 illustrates an example of transmitting a pushing ultrasound signal with respect to a plurality of focal points according to an embodiment of the present invention.

As shown in a block 401, the transceiving unit 111 may sequentially transmit the pushing ultrasound signal with respect to the plurality of focal points along a scan line. For example, the transceiving unit 111 may sequentially transmit the pushing ultrasound signal with respect to the focal points from A to I along a single scan line. Here, a frequency of the pushing ultrasound signal may be variable according to each of the focal points. For example, the transceiving unit 111 may transmit a relatively low frequency of pushing ultrasound signal with respect to a focal point with a relatively deep depth and thereby make the pushing ultrasound signal reaching a deep target tissue be less attenuated. Generally, the attenuation may incur more frequently as the frequency of the pushing ultrasound signal is higher and the depth of the focal point is deeper. Also, the transceiving unit 111 may transmit the tracking ultrasound signal with respect to the plurality of focal points along the scan line and receive a response signal reflected from the target tissue in correspondence to transmitting of the tracking ultrasound signal.

According to an embodiment of the present invention, when transmitting the pushing ultrasound signal to the target tissue via a plurality of scan lines, the transceiving unit 111 may simultaneously transmit the pushing ultrasound signal with respect to different focal points for each of the scan lines.

For example, as shown in a block 402, the transceiving unit 111 may apply the pushing ultrasound signal in a focal point order of A, B, and C via a first scan line, apply the pushing ultrasound signal in a focal point order of B, C, and A via a second scan line, and apply the pushing ultrasound signal in a focal point order of C, A, and B via a third scan line at the same time. Also, a frequency of the pushing ultrasound signal may be variable according to each of the different focal points. Displacement information associated with each of the different focal points may be simultaneously detected. Also, the transceiving unit 111 may simultaneously transmit the tracking ultrasound signal wit respect to the different focal points and receive a response signal reflected from the target tissue in correspondence to transmitting of the tracking ultrasound signal.

According to an embodiment of the present invention, when transmitting, to a target tissue, a pushing ultrasound signal for generating of a displacement and receiving a response signal from the target tissue in correspondence to transmitting of the tracking ultrasound signal, the transceiving unit 111 may use a probe including two-dimensionally arranged transducers. Here, the transducers may be classified into a plurality of sections. While transmitting the pushing ultrasound signal using a transducer included in a first section among the plurality of sections, the transceiving unit 111 may transmit the tracking ultrasound signal using a transducer included in a second section among the plurality of sections.

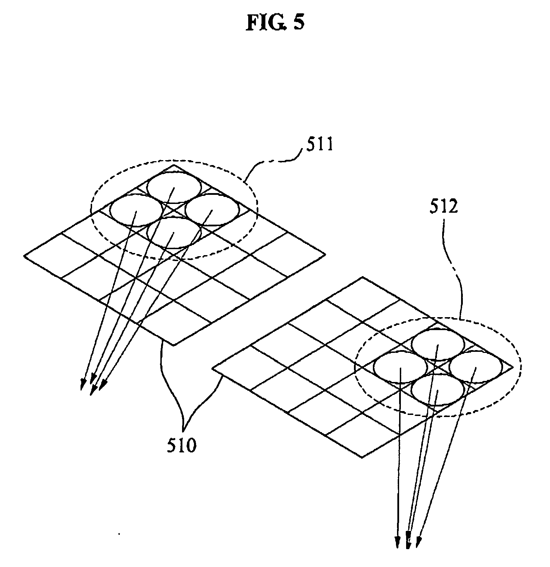

FIG. 5 illustrates an example of transmitting a different ultrasound signal for each transducer section according to an embodiment of the present invention.

Referring to FIG. 5, the transceiving unit 111 may transmit a pushing ultrasound signal using a transducer 511 included in a first section among a plurality of two-dimensionally arranged transducers 510 and simultaneously transmit a tracking ultrasound signal using a transducer 510 included in a second section. Also, while transmitting the pushing ultrasound signal using the transducer 511 of the first section, the transceiving unit 111 may receive a response signal using the transducer 512 of the second section. Also, while transmitting the pushing ultrasound signal to a first target tissue, a first ROI, or a first focal point using the transducer 511 of the first section, the transceiving unit 511 may transmit the pushing ultrasound signal to a second target tissue, a second ROI, or a second focal point using the transducer 512 of the second section.

According to an embodiment of the present invention, the transceiving unit 111 may use transducers, included in a particular section among the two-dimensionally arranged transducers 510 in order to generate a constant B mode ultrasound image. For example, the transceiving unit 111 may use an array corresponding to a bottom line among the arranged transducers 510 in order to generate the constant B mode ultrasound image. Specifically, the transceiving unit 111 may use transducers, included in a particular section, to transmit a constant tracking ultrasound signal and receive a response signal in correspondence thereto.

As described above, the transceiving unit 111 may allocate a different role to the transducers 510 for each section and thereby making it possible to more effectively obtain an acoustic radiation force impulse image.

Referring again to FIG 2, in operation S203, the detection unit 112 may detect displacement information associated with the target tissue using the response signal. Here, the response signal may include displacement information associated with the target tissue.

Specifically, the detection unit 112 may perform an envelope detection process that detects the magnitude of the response signal based on the response signal to thereby form ultrasound image data. Specifically, the detection unit 112 may form the ultrasound image data based on location information associated with a plurality of points existing in each scan line and data that is obtained from each of the points to thereby form ultrasound image data. Here, the ultrasound image data may include coordinates on an XY coordinate system at each point, angle information associated with each scan line with respect to a vertical scan line, data obtained at each point, and the like. Also, the detection unit 112 may compare ultrasound image data before and after a displacement of the target tissue occurs due to the applied pushing ultrasound signal and thereby may detect the displacement information.

In operation S204, the generation unit 113 may generate an acoustic radiation force impulse image based on the displacement information.

For example, the generation unit 113 may generate ultrasound image data associated with the target tissue or the ROI based on the response signal and generate a B mode ultrasound image using the ultrasound image data. Also, the generation unit 113 may generate the acoustic radiation force impulse image by overlapping the displacement information associated with the target tissue and the B mode ultrasound image.

Although not shown in FIG 2, the ultrasound system 110 may further perform transmitting a control command to a cooling device associated with the target tissue in correspondence to transmitting of the pushing ultrasound signal. Through this, the ultrasound system 110 may control a temperature rise of the target tissue caused by the pushing ultrasound signal. In particular, the ultrasound system 110 may operate the cooling device positioned on the epidermis of the target tissue, while transmitting the pushing ultrasound signal to the transducers of the probe.

The ultrasound method for providing the acoustic radiation force impulse image according to the above-described exemplary embodiments of the present invention may be recorded in computer-readable media including program instructions to implement various operations embodied by a computer. The media may also include, alone or in combination with the program instructions, data files, data structures, and the like. Examples of computer-readable media include magnetic media such as hard disks, floppy disks, and magnetic tape; optical media such as CD ROM disks and DVDs; magneto-optical media such as floptical disks; and hardware devices that are specially configured to store and perform program instructions, such as read-only memory (ROM), random access memory (RAM), flash memory, and the like. Examples of program instructions include both machine code, such as produced by a compiler, and files containing higher level code that may be executed by the computer using an interpreter. The described hardware devices may be configured to act as one or more software modules in order to perform the operations of the above-described exemplary embodiments of the present invention, or vice versa.

Although a few exemplary embodiments of the present invention have been shown and described, the present invention is not limited to the described exemplary embodiments. Instead, it would be appreciated by those skilled in the art that changes may be made to these exemplary embodiments without departing from the scope of the invention defined by the claims.

## Claims

1. An ultrasound method of providing an acoustic radiation force impulse image, the method comprising:
transmitting, to a target tissue (121), a pushing ultrasound signal for generating of a displacement (122), using a probe (114);
receiving a response signal from the target tissue (121) in correspondence to a tracking ultrasound signal transmitted via the probe (114);
detecting displacement information associated with the target tissue (121) using the response signal; and
generating the acoustic radiation force impulse image based on the displacement information, wherein the transmitting of the pushing ultrasound signal comprises simultaneously transmitting the pushing ultrasound signal along a plurality of scan lines that are spaced apart from each other by a predetermined distance, **characterized in that** the pushing ultrasound signal is transmitted with respect to different focal points for each of the scan lines, wherein a frequency of the pushing ultrasound signal is variable according to each of the different focal points.

2. An ultrasound method of providing an acoustic radiation force impulse image, the method comprising:
transmitting, to a target tissue (121), a pushing ultrasound signal for generating of a displacement, using a probe (114);
receiving a response signal from the target tissue (121) in correspondence to a tracking ultrasound signal transmitted via the probe (114);
detecting displacement information associated with the target tissue (121) using the response signal; and
generating the acoustic radiation force impulse image based on the displacement information, wherein the transmitting of the pushing ultrasound signal comprises sequentially transmitting the pushing ultrasound signal with respect to a plurality of focal points along a scan line, **characterized in that** a frequency of the pushing ultrasound signal is variable according to each of the focal points.

3. The method of claim 1 or 2, wherein the probe (114) includes two-dimensionally arranged transducers (510,511,512), the transducers are classified into a plurality of sections, and while the pushing ultrasound signal is being transmitted using a transducer (511) included in a first section among the plurality of sections, the tracking ultrasound signal is transmitted using a transducer (512) included in a second section among the plurality of sections.

4. The method of claim 1, 2 or 3, further comprising:
transmitting a control command to a cooling device associated with the target tissue (121) in correspondence to transmitting of the pushing ultrasound signal.

5. A computer-readable recording medium storing a program for implementing the method of any of claims 1 to 4 when executed on an ultrasound system according to claims 6 to 8.

6. An ultrasound system (110) for providing an acoustic radiation force impulse image adapted to operate according to an ultrasound method of any of claims 1 to 4, the system (110) comprising:
a transceiving unit (111) to transmit, to the target tissue (121), the pushing ultrasound signal for generating of the displacement, using a probe (114), and to receive the response signal from the target tissue (121) in correspondence to the tracking ultrasound signal transmitted via the probe (114);
a detection unit (112) to detect displacement information associated with the target tissue (121) using the response signal; and
a generation unit (113) to generate the acoustic radiation force impulse image based on the displacement information, wherein the transceiving unit (111) simultaneously transmits the pushing ultrasound signal along the plurality of scan lines that are spaced apart from each other by the predetermined distance or wherein the transceiving unit (111) sequentially transmits the pushing ultrasound signal with respect to the plurality of focal points along a scan line.

7. The system of claim 6, wherein the probe includes two-dimensionally arranged transducers (510,511,512), the transducers (510,511,512) are classified into a plurality of sections, and while the pushing ultrasound signal is being transmitted using a transducer (511) included in a first section among the plurality of sections, the tracking ultrasound signal is transmitted using a transducer (512) included in a second section among the plurality of sections.

8. The system of claim 6 or 7, further comprising:
a cooling device to decrease a temperature of the target tissue (121),
wherein the transceiving unit (111) transmits a control command to the cooling device in correspondence to transmitting of the pushing ultrasound signal.

## Patentansprüche

1. Ultraschallverfahren zur Bereitstellung eines akustischen Schallstrahlungs-Druckimpulsbilds, wobei das Verfahren Folgendes aufweist:
Übertragen eines aggressiven bzw. schiebenden Ultraschallsignals zu einem Zielgewebe (121) zum Erzeugen einer Verschiebung (122) unter Verwendung einer Sonde (114);
Empfangen eines Echosignals von dem Zielgewebe (121) in Übereinstimmung mit einem über die Sonde (114) übertragenen Verfolgungs-Ultraschallsignal;
Detektieren von Verschiebungsinformationen, die mit dem Zielgewebe (121) verbunden sind, unter Verwendung des Echosignals; und
Erzeugen des Schallstrahlungs-Druckimpulsbilds basierend auf den Verschiebungsinformationen, wobei das Übertragen des aggressiven Ultraschallsignals das gleichzeitige Übertragen des aggressiven Ultraschallsignals entlang einer Vielzahl von Scanlinien beinhaltet, welche um einen vorbestimmten Abstand voneinander entfernt sind,
**dadurch gekennzeichnet, dass**
das aggressive Ultraschallsignal unter Bezugnahme auf verschiedene Brennpunkte für jede der Scanlinien übertragen wird, wobei eine Frequenz des aggressiven Ultraschallsignals gemäß jedem der unterschiedlichen Brennpunkte variabel ist.

2. Ultraschallverfahren zur Bereitstellung eines akustischen Schallstrahlungs-Druckimpulsbilds, wobei das Verfahren Folgendes aufweist:
Übertragen eines aggressiven Ultraschallsignals zu einem Zielgewebe (121) zum Erzeugen einer Verschiebung unter Verwendung einer Sonde (114);
Empfangen eines Echosignals von dem Zielgewebe (121) in Übereinstimmung mit einem über die Sonde (114) übertragenen Verfolgungs-Ultraschallsignal;
Detektieren von Verschiebungsinformationen, die mit dem Zielgewebe (121) verbunden sind, unter Verwendung des Echosignals; und
Erzeugen des Schallstrahlungs-Druckimpulsbilds basierend auf den Verschiebungsinformationen, wobei das Übertragen des aggressiven Ultraschallsignals das sequentielle Übertragen des aggressiven Ultraschallsignals bezüglich einer Vielzahl von Brennpunkten entlang einer Scanlinie beinhaltet,
**dadurch gekennzeichnet, dass** eine Frequenz des aggressiven Ultraschallsignals gemäß jedem der unterschiedlichen Brennpunkte variabel ist.

3. Verfahren nach Anspruch 1 oder 2, wobei die Sonde (114) zweidimensional angeordnete Transducer (510,511,512) aufweist, wobei die Transducer in eine Vielzahl von Abschnitten eingeordnet sind, und wobei, während das aggressive Ultraschallsignal unter Verwendung eines Transducers (511) übertragen wird, der in einem ersten Abschnitt der Vielzahl von Abschnitten enthalten ist, das aggressive Ultraschallsignal unter Verwendung eines Transducers (512) übertragen wird, der in einem zweiten Abschnitt der Vielzahl von Abschnitten enthalten ist.

4. Verfahren nach Anspruch 1, 2 oder 3, welches des Weiteren Folgendes aufweist:
Übertragen eines Steuerbefehls an eine Kühlvorrichtung, die mit dem Zielgewebe (121) in Verbindung steht, in Übereinstimmung mit dem Übertragen des aggressiven Ultraschallsignals.

5. Computerlesbares Aufzeichnungsmedium, welches ein Programm zur Durchführung des Verfahrens nach einem der Ansprüche 1 bis 4 speichert, wenn es in einem Ultraschallsystem nach den Ansprüchen 6 bis 8 ausgeführt wird.

6. Ultraschallsystem (110) zur Bereitstellung eines Schallstrahlungs-Druckimpulsbilds, welches dafür vorgesehen ist, gemäß einem Ultraschallverfahren nach einem der Ansprüche 1 bis 4 betrieben zu werden, wobei das System (110) Folgendes aufweist:
eine Transceiving-Einheit (111), um das aggressive bzw. schiebende Ultraschallsignal zum Erzeugen der Verschiebung zu dem Zielgewebe (121) unter Verwendung einer Sonde (114) zu übertragen und um das Echosignal von dem Zielgewebe (121) in Übereinstimmung mit dem Verfolgungs-Ultraschallsignal zu empfangen, welches mittels der Sonde (114) übertragen wird;
eine Detektiereinheit (112), um Verschiebungsinformationen zu detektieren, die mit dem Zielgewebe (121) in Verbindung sind, unter Verwendung des Echosignals; und
eine Erzeugungseinheit (113) zum Erzeugen des Schallstrahlungs-Druckimpulsbilds basierend auf den Verschiebungsinformationen, wobei die Transceiving-Einheit (111) das aggressive Ultraschallsignal entlang der Vielzahl von Scanlinien gleichzeitig überträgt, welche um den vorbestimmten Abstand voneinander beabstandet sind, oder wobei die Transceiving-Einheit (111) das aggressive Ultraschallsignal bezüglich der Vielzahl von Brennpunkten entlang einer Scanlinie sequentiell überträgt.

7. System nach Anspruch 6, wobei die Sonde zweidimensional angeordnete Transducer (510,511,512) aufweist, wobei die Transducer (510,511,512) in eine Vielzahl von Abschnitten eingeordnet sind, und wobei, während das aggressive Ultraschallsignal unter Verwendung eines Transducers (511) übertragen wird, der in einem ersten Abschnitt der Vielzahl von Abschnitten enthalten ist, das aggressive Ultraschallsignal unter Verwendung eines Transducers (512) übertragen wird, der in einem zweiten Abschnitt der Vielzahl von Abschnitten enthalten ist.

8. System nach Anspruch 6 oder 7, welches des Weiteren Folgendes aufweist:
eine Kühlvorrichtung zum Verringern einer Temperatur des Zielgewebes (121), wobei die Transceiving-Einheit (111) einen Steuerbefehl an die Kühlvorrichtung in Übereinstimmung mit dem Übertragen des aggressiven Ultraschallsignals überträgt.

## Revendications

1. Procédé ultrasonore de production d'une image d'impulsions de force de radiation acoustique, le procédé comprenant :
l'émission, vers un tissu cible (121), d'un signal ultrasonore poussant pour produire un déplacement (122), en utilisant une sonde (114) ;
la réception d'un signal de réponse provenant du tissu cible (121) en correspondance avec un signal ultrasonore de poursuite émis par l'intermédiaire de la sonde (114) ;
la détection d'une information de déplacement associée au tissu cible (121) en utilisant le signal de réponse; et
la génération de l'image d'impulsions de force radiative acoustique sur la base de l'information de déplacement, dans lequel l'émission du signal ultrasonore poussant comprend l'émission simultanée du signal ultrasonore poussant le long d'une pluralité de lignes de balayage qui sont espacées l'une de l'autre d'une distance prédéterminée, **caractérisé en ce que** le signal ultrasonore poussant est émis pour différents foyers pour chacune des lignes de balayage, une fréquence du signal ultrasonore poussant variant avec chacun des différents foyers.

2. Procédé ultrasonore de production d'une image d'impulsions de force de radiation acoustique, le procédé comprenant :
l'émission, vers un tissu cible (121), d'un signal ultrasonore poussant pour générer un déplacement, en utilisant une sonde (114) ;
la réception d'un signal de réponse provenant du tissu cible (121) en correspondance avec un signal ultrasonore de poursuite émis par l'intermédiaire de la sonde (114) ;
la détection d'une information de déplacement associée au tissu cible (121) en utilisant le signal de réponse ; et
la génération de l'image d'impulsions de force de radiation acoustique sur la base de l'information de déplacement, dans lequel l'émission du signal ultrasonore comprend l'émission séquentiellement du signal ultrasonore poussant pour une pluralité de foyers le long d'une ligne de balayage, **caractérisé en ce qu'**une fréquence du signal ultrasonore poussant est variable selon chacun des foyers.

3. Procédé de la revendication 1 ou 2, dans lequel la sonde (114) comprend des transducteurs (510, 511, 512) disposés de façon bidimensionnelle, les transducteurs sont répartis en une pluralité de sections et, pendant que le signal ultrasonore poussant est émis en utilisant un transducteur (511) inclus dans une première section de la pluralité de sections, le signal ultrasonore de poursuite est émis en utilisant un transducteur (512) inclus dans une seconde section de la pluralité de sections.

4. Procédé de la revendication 1, 2 ou 3, comprenant en outre:
l'émission d'un ordre de commande à un dispositif de refroidissement associé au tissu cible (121) en correspondance avec l'émission du signal ultrasonore poussant.

5. Support d'enregistrement lisible par ordinateur, qui stocke un programme pour la mise en oeuvre du procédé de l'une quelconque des revendications 1 à 4, lorsqu'il est exécuté sur un système ultrasonore selon les revendications 6 à 8.

6. Système ultrasonore (110) pour fournir une image d'impulsions de force de radiation acoustique agencé pour mettre en oeuvre un procédé ultrasonore de l'une quelconque des revendications 1 à 4, le système (110) comprenant :
une unité d'émission-réception (111) pour émettre, vers le tissu cible (121), le signal ultrasonore poussant pour générer le déplacement, en utilisant une sonde (114), et pour recevoir le signal de réponse provenant du tissu cible (121) en correspondance avec le signal ultrasonore de poursuite émis par l'intermédiaire de la sonde (114);
une unité de détection (112) pour détecter une information de déplacement associée au tissu cible (121) en utilisant le signal de réponse ; et
une unité de génération (113) pour générer l'image d'impulsions de force de radiation acoustique sur la base de l'information de déplacement, dans laquelle l'unité d'émission-réception (111) émet simultanément le signal ultrasonore poussant le long de la pluralité de lignes de balayage qui sont espacées l'une de l'autre de la distance prédéterminée ou dans laquelle l'unité d'émission-réception (111) émet séquentiellement le signal ultrasonore poussant pour la pluralité de foyers le long d'une ligne de balayage.

7. Système de la revendication 6, dans lequel la sonde comprend des transducteurs disposés de façon bidimensionnelle (510, 511, 512), les transducteurs (510, 511, 512) sont répartis en une pluralité de sections et, pendant que le signal ultrasonore poussant est émis en utilisant un transducteur (511) inclus dans une première section de la pluralité de sections, le signal ultrasonore de poursuite est émis en utilisant un transducteur (512) inclus dans une seconde section de la pluralité de sections.

8. Système de la revendication 6 ou 7, comprenant en outre :
un dispositif de refroidissement pour réduire la température du tissu cible (121),
dans lequel l'unité d'émission-réception (111) émet un ordre de commande au dispositif de refroidissement en correspondance avec l'émission du signal ultrasonore poussant.
